# EUROPEAN PATENT APPLICATION

(11) **EP 1 454 988 A1**
(43) Date of publication of application: **08.09.2004**
(21) Application number: 03290504.4
(22) Date of filing: 03.03.2003
(51) Int. Cl.: C12N 15/86, C07K 14/18, C12N 15/40, G01N 33/569, A61K 39/12, A61K 48/00

(54) **Infectious flavivirus pseudo-particles containing functional prM-E envelope proteins**

(71) Applicant: INSTITUT NATIONAL DE LA SANTE ET DE LA RECHERCHE MEDICALE (INSERM), 75013 Paris (FR)
(72) Inventor: Bartosch, Birke, 38200 Chuzelles (FR); Cosset, Francois-Loic, 69007 Lyon (FR)
(74) Representative: Bernasconi, Jean Raymond

(57) **Abstract**

The invention relates to the generation and the use of flavivirus pseudo-particles containing native functional prM and E envelope glycoproteins assembled onto retroviral core particles. These particles are highly infectious and constitute a valid model of flavivirus virion whose handling does not require category 3 or 4 laboratories.

## Description

The invention relates to the generation and the use of flavivirus pseudo-particles containing native functional prM and E envelope glycoproteins assembled onto retroviral core particles. These particles are highly infectious and constitute a valid model of flavivirus virion whose handling does not require category 3 or 4 laboratories.

Flaviviruses are small, icosahedral enveloped viruses that constitute a genus within the family Flaviviridae, which also includes the genera pestivirus and hepacivirus (human hepatitis C viruses). Several flaviviruses are important mosquito-or tick-borne human pathogens, such as the yellow fever, dengue, Japanese encephalitis, West Nile and tick-borne encephalitis (TBE) viruses. Flavivirus structural proteins and non structural proteins are expressed from a single polyprotein precursor and individually released in their respective cell compartments upon cleavage by cellular and viral proteases. By analogy with other members of the Flaviviridae, flavivirus genomic organization suggests a virus consisting of a nucleocapsid comprising a viral genome and core protein (C) coated by a lipid envelop containing the two envelope glycoproteins prM and E. The E protein is the major constituent of the virion surface and has the dual function of binding cell receptors and mediating lowpH-triggered membrane fusion in the endosome. Virus assembly takes place in the endoplasmic reticulum and first leads to the generation of fusion-incompetent, immature virions in which the E protein forms a stable heterodimeric complex with precursor of M (prM). Immature virions are transported through the cellular endocytotic pathway and, shortly before their release, prM is cleaved by furin or a related protease in the trans-Golgi network to generate fusion-competent mature infectious virions.

West Nile virus (WNV), a single-stranded positive-polarity RNA virus, is the etiologic agent of West Nile encephalitis. WNV is maintained in a natural cycle between mosquitoes and birds but also infects humans, horses, and other animals. It is endemic in parts of Africa, Europe, the Middle East, and Asia (Hubalek and Halouzka, 1999), and outbreaks in the United States over the past 3 years indicate that it has established its presence in the Western Hemisphere (Lanciotti et al., 1999). Humans develop a febrile illness, with a subset of cases progressing to a meningitis or encephalitis syndrome (Hubalek and Halouzka, 1999). Currently, no specific therapy or vaccine has been approved for human use.

The molecular basis of WNV infection in humans and other animals is not clearly established. Although prior rodent models of WNV infection have shown evidence of viral replication in serum and the central nervous system (CNS), many of the mechanistic questions about pathogenesis and the immune response remain unanswered. For example, a peripheral site of replication has not been defined and the mechanism of spread to the CNS remains unclear. Additionally, a target cell for infection in the CNS has not been definitively identified, and whether tissue injury is related directly to viral infection or to the immune response remains unknown. Finally, although susceptibility to severe WNV infection correlates with an impaired immune system (Hubalek and Halouzka, 1999), the mechanisms by which the innate and adaptive immune responses limit disease have not been established. Experiments with related and unrelated RNA viruses have suggested that antibody may have an important protective role against WNV infection. Passive administration of monoclonal antibodies (MAbs) limits the encephalitis caused by some flaviviruses (Saint Louis encephalitis, Japanese encephalitis, and yellow fever viruses) and nonflaviviruses (Sindbis, murine hepatitis, and lymphocytic choriomeningitis viruses). However, for many of these viruses the mechanism by which antibody attenuates CNS infection has not been clearly demonstrated. The in vitro properties of MAbs, including isotype, avidity, and neutralization, do not necessarily correlate with protection, as antibodies may limit viral infection through different mechanisms at several stages of pathogenesis. Antibody may decrease viral load in the CNS by limiting hematogenous spread through direct neutralization, complement activation, or increased viral uptake by phagocytic cells. Alternatively, antibodies may act directly in the CNS by preventing replication and spread in neurons.

The invention describes the formation and the use of infectious flavivirus pseudo-particles harboring unmodified prM and E glycoproteins.

### Definitions

The terms *"vector", "cloning vector"* and *"expression vector"* mean the vehicle by which a DNA or RNA sequence (e.g. a foreign gene) can be introduced into a host cell, so as to transform the host and promote expression (e.g. transcription and translation) of the introduced sequence. Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a *"DNA construct".* A common type of vector is a *"plasmid",* which generally is a self-contained molecule of double-stranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts.

A *"coding sequence"* or a sequence *"encoding"* an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme.

The term *"transfection"* means the introduction of a foreign nucleic acid (DNA, cDNA or RNA) into a cell so that the host cell will express the introduced gene or sequence to produce a desired substance, typically a protein coded by the introduced gene or sequence. The introduced gene may include regulatory or control sequences, such as start, stop, promoter, signal, secretion, or other sequences used by a cell's genetic machinery. A host cell that receives and expresses introduced DNA or RNA has been *"transformed".*

The term *"host cell"* means any cell of any organism that is selected, modified, transformed, grown, or used or manipulated in any way, for the production of a substance by the cell, for example the expression by the cell of a gene, a DNA sequence, a protein, a virion. In the context of the invention, the host cell is a mammalian cell. In particular, the host cell may be selected from the group consisting of mononuclear macrophages, monocytes or dendritic cells, endothelial cells, fibroblasts, neurone/neuroblastoma cells, hepatocyte/hepatoma cells. Suitable host cells include 293T human embryo kidney cells (ATCC CRL-1573), Huh-7 human hepatocellular carcinoma (Nakabayashi et *al.,* 1982), Hela human epithelial adenocarcinoma (CCL-2), PS, LLMK2, SW13, Neuro-2a mouse neuroblastoma (CCL-131), HepG2 human hepatocellular carcinoma (HB-8065), TE671 human rhabdomyosarcoma (CRL-8805), Vero African green monkey kidney (CCL-81), and BHK-21 golden hamster kidney (CCL-1 0).

As used herein, the term *"permissive cell"* is meant for a cell that is permissive for flavivirus infection. Examples of permissive cells include the above-mentioned mammalian mononuclear macrophages, monocytes or dendritic cells, endothelial cells, fibroblasts, neurone/neuroblastoma cells, hepatocyte/hepatoma cells. *"Flavivirus"* is a genus of the Flaviviridae virus family which also includes pestivirus and hepacivirus. Flavivirus genome encodes a single polyprotein NH2-C-prM-E-NS1-NS2a-NS2b-NS3-NS4a-NS4b-NS5-COOH that is processed co and post-translationally into both structural (N-terminal nucleocapsid protein termed "Core" (C), and glycoproteins prM and E) and non-structural (NS) proteins. In the context of the invention, flavivirus is of any member, genotype, strain, subtype and variants thereof.

The term *"variant"* refers to the homologous polynucleotide sequences and corresponding amino acid sequences found in the different flavivirus strains owing to flavivirus hypervariability.

Tick borne viruses such as tick-borne encephalitis virus or Louping ill virus, and Mosquito-borne viruses such as Dengue Virus (DEN), Yellow Fever Virus (YFV), West Nile Virus (WNV), or japanese encephalitis virus, are examples of flaviviruses.

Preferably, said flavivirus is selected from the group consisting of West Nile Virus (WNV), Dengue Virus (DEN) or Yellow Fever Virus (YFV). Preferably said flavivirus is a West Nile Virus (WNV), still preferably the Israelian strain ISR98-G001). Preferably said flavivirus is a Dengue Virus (DEN), still preferably from the DEN-1 (Fga/89) or from the DEN-2, DEN-3 and DEN-4 strains. Preferably said flavivirus is a Yellow Fever Virus (YFV), still preferably from the 17D-204-Pasteur strain.

The term *"flavivirus-like particles"* or *"flavivirus pseudo-particles"* as used herein refers to non naturally occurring viral particles that comprise an envelope protein of flavivirus. The flavivirus pseudo-particles of the invention are infectious for a target cell. The particles of the invention more particularly comprise retroviral core proteins. Such particles may be readily produced by one skilled in genetic engineering techniques. One can for instance refer to EP 1 201 750 that describes production of synthetic retroviral particles expressing an antigen for modulating an immune response.

In the context of the invention, the term *"infectious"* is used to describe the capacity of the particles of the invention to complete the initial steps of viral cycle that lead to cell entry. However, upon interaction with the host cell, flavivirus-like particles may or may not produce progeny viruses.

The term *"an envelope protein of flavivirus"* denotes the native prM or E glycoprotein of flavivirus, or a mutant thereof.

By an *"prM glycoprotein"* or *"prM protein"* is meant an envelope prM protein from any genotype, subtype and variant of flavivirus strains.

By an *"E glycoprotein"* or *"E protein"* is meant an envelope E protein from any genotype subtype and variant of flavivirus strains.

Preferably, prM and E glycoproteins are derived from a same flavivirus strain.

The term *"mutant"* or *"mutation"* is meant for alteration of the DNA sequence that result in a modification of the amino acid sequence of native prM or E proteins. Such a modification can be for instance the substitution and/or deletion of one or more amino acids. Mutants notably include fragments of native prM and E proteins. Variants are particular examples of naturally occurring mutants. Mutants are more particularly contemplated as useful for identifying the structural elements of prM and/or E proteins necessary for maintaining cell infectivity or for increasing prM and/or E antigenicity for vaccination purposes.

As used herein, the term *"flavivirus core"* denotes a native core protein of the various flavivirus strains, a fragment thereof, or a variant thereof. The flavivirus core provides a signal peptide for the prM or optionally E linked thereto that allows protein translocation to the endoplasmic reticulum. West Nile virus core signal peptide corresponds to the last 22 residues of the carboxy-terminus of WNV core (QKKRGGKTGIAVMIGLIASVGA, SEQ ID No1). Dengue virus core signal peptide corresponds to the last 20 residues of the carboxy-terminus of DEN-1 core (MNRRKRSVTMLLMLLPTVLA, SEQ ID No2). Yellow fever virus core signal peptide corresponds to the last 20 residues of the carboxy-terminus of YFV core (RSHDVLTVQFLILGMLLMTG, SEQ ID No 3). According to an embodiment, the core protein is thus a N-terminally truncated form of flavivirus core (ΔC) that comprises the flavivirus core signal peptide. Preferably ΔC comprises the last 20, preferably the last 22, residues of the carboxy-terminus of flavivirus core. In particular, flavivirus core may consist in the last 60 residues of the carboxy-terminus of flavivirus core.

In the context of the invention, the terms *"native"* or *"unmodified"* are indifferently used to describe a wild-type, full-length protein.

The term *"polyprotein"* as used herein is used to describe a protein construct made up of individual proteins that are joined together in a sequence whereby they retain their original relevant biological activities.

The term *"a polyprotein comprising a flavivirus core protein linked to flavivirus prM protein and*/*or flavivirus E protein"* or "*a polyprotein comprising successively a flavivirus core protein, and a flavivirus prM protein and*/*or a flavivirus E protein"* includes the CprME, CEprM, CprM, CE, ΔCprME, ΔCEprM, ΔCprM, and ΔCE polyproteins. *"CprME"* denotes a polyprotein comprising successively a flavivirus core protein, a flavivirus prM protein and a flavivirus E protein. *"CEprM"* denotes a polyprotein comprising successively a flavivirus core protein, a flavivirus E protein and a flavivirus prM protein. *"CprM"* denotes a polyprotein comprising a flavivirus core protein linked to a flavivirus prM protein. "*CE*" denotes a polyprotein comprising a flavivirus core protein linked to a flavivirus E protein. *"ΔCprME"* denotes a polyprotein comprising successively a carboxy terminus of flavivirus core protein, and flavivirus prM and flavivirus E proteins. *"ΔCEprM"* denotes a polyprotein comprising successively a carboxy terminus of flavivirus core protein, and flavivirus E and flavivirus prM proteins. *"ΔCprM"* denotes a polyprotein comprising a carboxy terminus of flavivirus core protein linked to a flavivirus prM protein. *"ΔCE"* denotes a polyprotein comprising a carboxy terminus of flavivirus core protein linked to a flavivirus E protein. ΔCprME, as well as ΔCE, have been built by inserting a stop codon at the end of E, whereas ΔCEprM and ΔCprM have been built by inserting a stop codon at the end of prM.

By *"retrovirus"* is meant a virus whose genome consists of a RNA molecule and that comprises a reverse-transcriptase, *i.e.* a member of the Retroviridae family. Retroviruses are divided into Oncovirus, Lentivirus and Spumavirus. Preferably said retrovirus is an oncovirus, e.g. MLV, ALV, RSV, or MPMV, a lentivirus, e.g. HIV-1, HIV-2, SIV, EIAV, or CAEV, or a spumavirus such as HFV. Genomes of these retroviruses are readily available in databanks.

In the context of the invention *"a nucleic sequence comprising a packaging competent retrovirus-derived genome"* is intended for a sequence that comprises the retroviral nucleic acid sequences known as "cis-acting" sequences. These include the Long Terminal Repeats (LTRs) for the control of transcription and integration, the psi sequence necessary for encapsidation, and the Primer Binding site (PBS) and polypurine track (PPT) sequences necessary for reverse transcription of the retroviral genome. Advantageously, said nucleic acid sequence comprising a packaging competent retrovirus-derived genome further comprises a transgene.

Said retroviral genome may be replication-defective or replication-competent, in the absence of any trans-complementing function. A replication-competent genome would further comprise the gag, pol, and env retroviral genes. In a replication-defective genome, the viral genes gag, pol, and env are deleted. However, assembly of viral pseudo-particles may be achieved by providing another vector that comprises gag, pol and env but that is defective for the "cis" sequences. Their expression allows the encapsidation of the transgene, excluding the genes necessary for the multiplication of the viral genome and for the formation of complete viral particles.

As used herein, the term *"transgene"* designates the gene that is expressed in the target cell upon infection by the particles of the invention.

Examples of transgenes include a gene encoding a molecule of therapeutic interest, a marker gene, a gene coding for an immune modulator, an antigen, or a suicide gene.

A *"marker gene"* denotes a gene whose expression is detectable. For instance marker gene expression can generate a detectable signal, such as a fluorescence emission, a chromogenic reaction, or confer a growth advantage to the cells wherein it is expressed (antibiotic resistance genes).

An *"immune modulator"* refers to the product of a gene that modifies the activity of the immune system of a subject *in vivo.* Examples of immune modulators include cytokines, (*e.g*. interleukins, interferons, or haematopoietic colony stimulating factors), chemokines, and the like. Expression of an immune modulator by transformed cells may change the cellular environment and alter differentiation of immune cells and thus modify the type and the strength of immune response elicited against a given antigen.

An *"antigen"* refers to a molecule, such as a peptide, a polypeptide or a protein, against which an immune response is sought. Said antigen may be for instance a tumor, a bacterial, a pathogenic, a proteic, or a viral antigen.

A *"suicide gene"* is meant for a gene whose expression in cells induces programmed-cell death (apoptosis) such as the conditional Herpes Simplex virus type I thymidine kinase gene.

The *"core protein from a retrovirus"* refers to proteins encoded by the gag and pol genes. The gag gene encodes a polyprotein which is further processed by the retroviral protease into structural proteins that comprise the core. The pol gene encodes the retroviral protease, reverse-transcriptase, and integrase.

A *"pharmaceutically acceptable carrier"* refers to any vehicle wherein the vaccine composition according to the invention may be formulated. It includes a saline solution such as phosphate buffer saline. In general, a diluent or carrier is selected on the basis of the mode and route of administration, and standard pharmaceutical practice.

In the context of the present application, *"vaccination"* is intended for prophylactic or therapeutical vaccination. *"Therapeutical vaccination"* is meant for vaccination of a patient with flavivirus infection.

According to the invention, the term *"subject"* or *"patient"* is meant for any mammal, human or animal, likely to be infected with flaviviruses.

### Production of flavivirus particles

The inventors have generated infectious pseudo-particles that contain functional, and more particularly unmodified, flavivirus glycoproteins assembled onto retroviral core particles. Flavivirus prME are expressed from a polyprotein containing the core (C) protein or a fragment thereof, in particular the carboxy-terminus of the C protein, which served as signal peptide for prM or E, and the prM and/or E glycoproteins.

The invention thus provides a method for producing flavivirus (FV)-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a flavivirus core protein, and a flavivirus prM protein and/or a flavivirus E protein;
- transfecting host cells with said nucleic acid sequences and maintaining the transfected cells in culture for sufficient time to allow expression of the cDNAs to produce structural proteins from flavivirus and retrovirus; and allowing the structural proteins to form virus-like particles.

The invention further provides a method for producing flavivirus (FV)-like particles *in vivo,* which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a flavivirus core protein, and a flavivirus prM protein and/or a flavivirus E protein;
- transfecting cells of a subject *in vivo* with said nucleic acid sequences, to allow expression of the cDNAs to produce structural proteins from flaviviruses and retrovirus; and to allow the structural proteins to form virus-like particles.

Another aspect of the invention is the use of three nucleic acid sequences for the preparation of a medicament useful as a vaccine against a flavivirus, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a flavivirus core protein, and a flavivirus prM protein and/or a flavivirus E protein ;
and, when transferred into cells of a subject, the nucleic acid sequences allow the production of structural proteins from flaviviruses and retrovirus, wherein the structural proteins form virus-like particles that are immunogenic.

For the purpose of transfection, said first, second and third nucleic acid sequences may be carried on a same vector, or on two or three separated vectors.

In particular, plasmoviruses, adenoretroviruses and replicating pseudo-viruses are examples of vectors suitable for carrying the above-mentioned sequences. A plasmovirus vaccine consists in such a plasmid DNA preparation, that allow expression of flavivirus pseudo-particles after administration in an patient in order to elicit a immune response against said flavivirus. Administration of such a plasmovirus vaccine being achieved for preventive vaccination into people at risk for flavivirus-induced disease or for therapeutic vaccination into flavivirus-infected patients. Adenoretroviruses consist in an alternative way to provide the above-mentioned nucleic acid sequences encoding flavivirus pseudo-particles. In this case, it is possible to design three independent adenoretroviruses, *i.e.* recombinant adenoviruses, that encode the three nucleic acid sequences mentioned above (retroviral core and genome and flavivirus glycoproteins), or, alternatively, it is also possible to design a single adenoretrovirus, derived from "guttless" recombinant adenoviruses, that contains the different nucleic acid sequences. Such adenoretroviruses can be administered to patient as for plasmoviruses, in order to elicit an anti-flavivirus immune response. Replicating pseudo-retroviruses are another alternative possibility to express all the above-mentioned nucleic acid sequences encoding the flavivirus pseudo-particles. Such structures are in fact flavivirus-pseudo-particles whose genome is engineered to allow, following infection, its propagation into cells of an inoculated patient, thereby inducing the production of further replicating flavivirus pseudo-particles. In this case the genome of a retrovirus is modified so as to express the flavivirus E and prM glycoproteins in place of the retroviral Env gene (encoding the retroviral glycoproteins). The genes encoding the retroviral core proteins are left unchanged. Futhermore an additional gene, encoding a marker gene or an immunomodulator, for example, can be expressed from this genome.

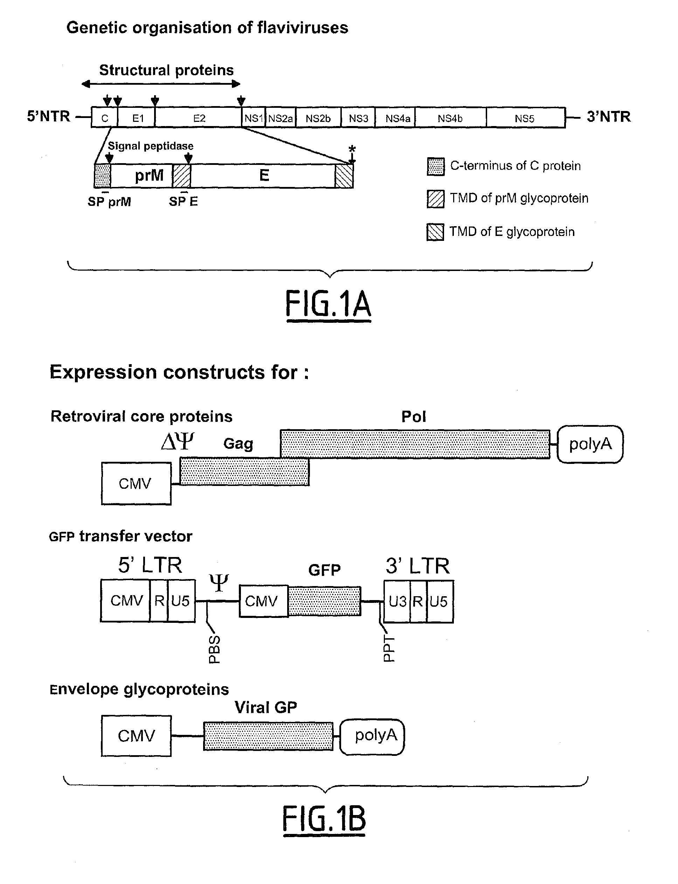

An example of flavivirus prME and retroviral expression constructs is shown in figures 1A and 1B. Preferably, said flavivirus is selected from the group consisting of West Nile Virus, Dengue Virus and Yellow Fever Virus.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator.

In the method of the invention, said polyprotein may comprise a flavivirus core protein linked to a flavivirus prM protein, or a flavivirus core protein linked to a flavivirus E protein, or successively a flavivirus core protein, a flavivirus prM protein and a flavivirus E protein, or successively a flavivirus core protein, a flavivirus E protein and a flavivirus prM protein.

According to an embodiment, prM and/or E are native proteins. According to another embodiment, prM and/or E glycoproteins are mutated to obtain particles that are useful for characterizing the glycoprotein determinants for flavivirus infectivity.

Preferably, said prM and E glycoproteins are both derived from a same flavivirus strain.

According to another embodiment said flavivirus core protein is a carboxy terminus form (ΔC) of flavivirus core protein that comprises the core protein signal peptide. In particular, said flavivirus core protein may comprise the last 20, preferably the last 22, amino acids of the carboxy-terminus of flavivirus core.

In accordance with the present invention there may be employed conventional molecular biology, microbiology, and recombinant DNA techniques within the skill of the art. Such techniques are explained fully in the literature. See, e.g., Sambrook et al., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et *al.,* 1994.

In particular, the vectors of the invention may be introduced into the target cell by means of any technique known for the delivery of nucleic acids to the nucleus of cells, either in culture, *ex vivo,* or *in vivo.*

Introduction of the nucleic acid sequences may be performed by any standard method well known by one skilled in the art, e.g. transfection, electroporation, microinjection, transduction, cell fusion, DEAE dextran, calcium phosphate precipitation, or use of a gene gun (see for instance Wu et al., 1992 ; Wu et al, 1988).

The donor nucleic acid targeting system can also be introduced by lipofection. In certain embodiments, the use of liposomes and/or nanoparticles is contemplated for the introduction of the donor nucleic acid targeting system into host cells. Nanocapsules can generally entrap compounds in a stable and reproducible way. Ultrafine particles (sized around 0.1 µm) that can be designed using biodegradable polyalkyl-cyanoacrylate polymers are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 A, containing an aqueous solution in the core. The use of cationic lipids may promote encapsulation of negatively charged nucleic acids, and also promote fusion with negatively charged cell membranes (Felgner et al., 1989).

*In vivo* targeted gene delivery is described in international patent publication WO 95/28 494. Alternatively, the vector can be introduced *in vivo* by lipofection, using liposomes or nanoparticles as above described. It is also possible to introduce the vector *in vivo* using techniques that are similar to the techniques that are employed *in vitro (e.g.* transfection, electroporation...).

### Transformed cells

The invention further relates to a transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a flavivirus core protein, and a flavivirus prM protein and/or a flavivirus E protein.

Such a transformed host cell is obtainable as described in a method above.

In another aspect, the invention relates to the use of a transformed host cell as defined above, for the identification of molecules capable of interfering with flavivirus entry in cells. The invention provides in particular a method of ex vivo screening or identification of molecules capable of interfering with flavivirus entry in cells comprising comparison of the level of transformed host cell fusion to a target host cell, in the presence or the absence of a candidate molecule. Said method preferably comprises the steps consisting of:
- co-culturing a transformed host cell with a target host cell, in the absence or presence of a candidate molecule, under conditions that allow syncytia formation, *i.e.* cell-cell fusion, and flavivirus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with flavivirus entry the candidate molecule for which syncytia formation, as measured in the presence of said molecule, is decreased as compared to syncytia formation measured in the absence of any candidate molecule.

Contacting a transformed host cell with a target host cell, and a candidate molecule can be carried out by contacting simultaneously said transformed host cell, target host cell and candidate molecule. Otherwise, two of these three elements can be contacted under conditions sufficient to allow their interaction before addition of the third missing element.

Preferably said target host cell is not transformed, *i.e.* said target host cell does not contains at least one of the first, second, and third nucleic acid sequence as defined above.

Syncytia formation can be readily assessed by one skilled in the art. Briefly, the coculture is submitted to a acidic pH drop by incubation for 5 min at pH-5 and incubated in a nomal medium for an additional 12 hrs. Cultures are then stained by adding the May-Grunwald and Giemsa solutions (MERCK) according to the manufacturer recommendations. Cells containing two or more nuclei can be defined as syncytia. A fusion index is then defined as the percentage of (N-S)/T where N is the number of nuclei in the syncytia, S is the number of syncytia and T is the total number of nuclei counted.

### Flavivirus-like particles

In the method described above no structural modifications of the prME glycoproteins are required for their correct assembly on retroviral cores. The method of the invention thus makes it possible to generate high titre infectious flavivirus pseudo-particles with functional prME glycoproteins. As demonstrated herein, these particles constitute a valid model of flavivirus virion as regards to early steps of viral infection cycle.

The invention further relates to an infectious flavivirus-like particle, comprising the core proteins from a retrovirus, and prM and/or E flavivirus glycoprotein(s). Such a particle is obtainable by a method as described above.

According to an embodiment, the infectious particle of the invention may comprise native flavivirus prM protein, native flavivirus E protein, or native flavivirus prM protein and native flavivirus E protein. Preferably said prM and E glycoproteins are both derived from a same flavivirus strain. According to another embodiment, prM and/or E glycoproteins are mutated.

Said retrovirus may be selected from the group consisting of MLV, ALV, RSV, MPMV, HIV-1, HIV-2, SIV, EIAV, CAEV, and HFV.

Advantageously, said infectious particles further carry a transgene. For instance said transgene may be a marker gene which make it possible to follow-up cell infection by the infectious particles of the invention and can find application for instance in the identification of a cell receptor involved in flavivirus entry. Said transgene can also be a gene encoding a molecule of therapeutic interest and/or a suicide gene. Accordingly, the particles of the invention that specifically target mammalian mononuclear macrophages, monocytes or dendritic cells, endothelial cells, fibroblasts, neurone/neuroblastoma cells, hepatocyte/hepatoma cells comprise a useful vector for gene transfer and/or gene therapy.

### Use of the infectious flavivirus-like particles of the invention

High infectivity of these particles makes it possible for the investigation of the role of flavivirus prM and E glycoproteins and their potential receptors in cell entry, flavivirus host-range and neutralisation by antibodies from flavivirus patient sera.

The invention therefore concerns the use of a flavivirus-like infectious particle as described above, for *ex vivo* identification of a cell receptor for flavivirus prM and/or E glycoprotein.

According to an embodiment, the invention provides a method for *ex vivo* identification of a receptor for flavivirus prM and/or E glycoprotein comprising detection of the binding of said particle to a cell receptor. More specifically, the method may comprise the steps consisting of:
- contacting a cell susceptible to flavivirus infection with an infectious flavivirus-like particle of the invention, under conditions sufficient to allow specific binding of said particle to a receptor expressed at the surface of said cell;
- detecting binding of said particle to a receptor; and
- identifying said receptor.

A cell susceptible to flavivirus infection may preferably be selected from the group consisting of mammalian mononuclear macrophages, monocytes or dendritic cells, endothelial cells, fibroblasts, neurone/neuroblastoma cells, hepatocyte/hepatoma cells.

Detection of particle binding to a receptor can be achieved according to classical procedures well-known by one skilled in the art. For instance, this could involve radioactive, enzyme or fluorescent labelling of the particles of the invention, and subsequent detection with an appropriate method. A number of fluorescent materials are known and can be utilized as labels. These include, for example, fluorescein, rhodamine, auramine, Texas Red. Enzyme labels consist in conjugation of an enzyme to a molecule of interest, e.g. a polypeptide, and can be detected by any of colorimetric, spectrophotometric, or fluorospectrophotometric techniques. Flow cytometry analysis (FACS) together with labelled antibodies directed against prM or E proteins harboured by the pseudo-particles of the invention is also appropriate.

According to another embodiment, the invention provides a method for *ex vivo* identifying a cell receptor for flavivirus comprising the step consisting of:
- transfecting a cell which is not permissive for flavivirus infection with a nucleic acid sequence encoding a protein likely to be a flavivirus receptor;
- contacting said transformed cell with a flavivirus-like particle of the invention;
- determining whether said transformed cell has become permissive or not for flavivirus infection; and
- identifying as a cell receptor for flavivirus said protein expressed by the transformed cell that has become permissive.

Determination of whether the transformed cell has become permissive for flavivirus infection can be readily achieved using the flavivirus-like particles of the invention. In particular, where said particles carry a marker gene, such as GFP, permissivity *(i.e.* the capacity of cells to be infected with flaviviruses, and in particular with flavivirus-like particles) can be assessed by FACS analysis of the transformed cells. Where the marker gene is an antibiotic resistance gene, identification of cells infected by flavivirus-like particle is readily achieved through exposure to said antibiotic.

Where one does not suspect a given protein to be a receptor for flavivirus entry in cells, the above method can advantageously be adapted for the screening and the identification of a cell receptor for flaviviruses. In particular, an expression cDNA library can be prepared, for instance from a cDNA library obtained by reverse-transcription of cellular mRNAs from a cell permissive for flavivirus infection. Expression of such a cDNA library would be driven by a constitutive promoter whose nucleic acid sequence has been fused to the cDNA library in suitable vectors. Such a library would contain a vector encoding a cell receptor for flaviviruses. Non permissive cells can then be transfected with this expression library and further screened for the identification of a cell receptor for flaviviruses.

To this end, the invention proposes a method for *ex vivo* identifying a cell receptor for flavivirus comprising the step consisting of:
- providing an expression cDNA library obtained from a cell permissive for flavivirus infection;
- transfecting cells that are not permissive for flavivirus infection with said expression cDNA library;
- contacting said transformed cells with flavivirus-like particles of the invention;
- identifying and isolating those transformed cells that have become permissive for flavivirus infection;
- isolating the expression vector transfected in cells that have become permissive; and
- identifying as a receptor for flavivirus the proteins encoded by the cDNA sequence of said isolated expression vectors.

Determination of whether the transformed cell has become permissive for flavivirus infection can be readily achieved using the flavivirus-like particles of the invention. In particular, where said particles carry a marker gene, such as GFP, permissivity *(i.e.* the capacity of cells to be infected with flaviviruses, and in particular with flavivirus-like particles) can be assessed by FACS analysis of the transformed cells. Where the marker gene is an antibiotic resistance gene, identification of cells infected by flavivirus-like particle is readily achieved through exposure to said antibiotic.

Advantageously, the expression cDNA library is expressed from retroviral vectors that comprises glycoproteins that allow infection of the flavivirus non permissive cells. Such glycoproteins can be the VSV-G glycoprotein derived from vesicular stomatitis virus (VSV) whose receptor is expressed in most cell types *ex vivo.* Such viral particles can be assembled using a packaging competent retrovirus-derived genome that comprises the expression cDNA library, and optionally a marker gene. According to this embodiment the method for isolating the expression vector expressed in cells that have become permissive to infection by the flavivirus-like particles of the invention is greatly facilitated. Indeed this latter embodiment is particularly advantageous in that the process of cell infection with retroviral vectors has greater efficacy, as compared to cell transfection. Furthermore, cell infection leads to stable integration of viral genome in the cellular genome. Accordingly, transgenes, i.e. cDNA and marker gene that are carried by the pseudo-particles of the invention, are found to be stably expressed by infected cells. This in contrast with classical vectors used for transfection that do not integrate into cellular genome and for which expression may be transient.

In another aspect, the invention relates to the use of an infectious particle as defined above, for the identification of molecules capable of interfering with flavivirus entry in cells. In particular, herein is provided a method of *ex vivo* screening or identification of molecules capable of interfering with flavivirus entry in cells comprising comparison of the level of cell infection by the particles of the invention in the presence or the absence of a candidate molecule. Said method preferably comprises the steps consisting of:
- contacting a cell susceptible to flavivirus infection with an infectious flavivirus-like particle, in the absence or presence of a candidate molecule, under conditions that allow cell infection with flavivirus-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with flavivirus entry the candidate molecule for which cell infectivity, as measured in the presence of said molecule, is decreased as compared to cell infectivity measured in the absence of any candidate molecule.

Contacting a cell susceptible to flavivirus infection with an infectious flavivirus-like particle, and a candidate molecule can be carried out by contacting simultaneously said cell, flavivirus-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious flavivirus-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with flavivirus cell entry may be any permissive cell for flavivirus infection, as described above.

Such molecules capable of interfering with flavivirus entry in cells may constitute new antiviral drugs.

The infectious particles of the invention are further useful for diagnosis of flavivirus infection and follow-up of flavivirus infection, for instance to assess efficacy of a therapy in a patient.

The invention thus concerns the use of an infectious flavivirus-like particle for the *in vitro* detection of antibodies directed against flavivirus in a biological sample from a subject susceptible to be infected with flaviviruses. Said biological sample may be a biological fluid, such as blood or serum, or a tissue biopsy. In a specific embodiment, said antibodies are directed against prM and/or E flavivirus glycoproteins.

Accordingly, the invention provides a method of *in vitro* diagnosis of a flavivirus infection in a patient comprising detecting immune complexes formed by interaction of anti-flavivirus antibodies likely to be present in a biological sample of the patient, with flavivirus-like particle of the invention. Said method may in particular comprise the steps consisting of:
- contacting a biological sample with an infectious flavivirus-like particle of the invention under conditions sufficient to allow formation of complexes by binding of said infectious particle to antibodies directed against flavivirus present in the biological sample;
- detecting said complexes, which presence is indicative of a flavivirus infection.

The presence of antibodies reactive with flavivirus-like particles can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the flavivirus-like particle and the antibody or antibodies reacted therewith.

In another embodiment, said method of *in vitro* diagnosis of a flavivirus infection in a patient comprises detecting an inhibitory effect of anti-flavivirus antibodies likely to be present in a biological sample of the patient, on the infection of a permissive cell by a flavivirus-like particle of the invention. Said method may in particular comprise the steps consisting of:
- contacting a cell permissive for flavivirus infection with a flavivirus-like particle and a biological sample;
- comparing cell infectivity measured in presence of said biological sample with cell infectivity measured in absence of said biological sample;
- detecting the inhibition of flavivirus-like particle infection of a permissive cell as a decrease in cell infectivity measured in presence of said biological sample compared with cell infectivity measured in absence of said biological sample, said inhibition being indicative of a flavivirus infection.

This embodiment is advantageous in that the method relies on the detection of the specific antibodies that are neutralizing for cell infection, that is those patient's antibodies that are effective against viraemia.

In a further embodiment of this invention, commercial diagnostic kits may be useful to carry out the above diagnosis methods, by detecting the presence or absence of immune complexes formed by flavivirus particles and antibodies directed against flavivirus in a biological sample from a subject susceptible to be infected with flaviviruses, or by detecting an inhibition of flavivirus-like particle infection of a permissive cell by anti-flavivirus neutralizing antibodies likely to be present in a biological sample of the patient. Such kits may comprise at least a flavivirus-like particle of the present invention. Where the method involves detection of immune complexes, the kits may further comprise appropriate means of detection of said immune complexes. Preferably the kit of the invention further comprises directions, and protocols, depending upon the method selected, *e.g*., "competitive", "sandwich", and the like. The kits may also contain peripheral reagents such as buffers, stabilizers, etc..

In another aspect of the invention, the infectious flavivirus-like particles may be used for vaccination purposes.

According to an embodiment, the invention thus proposes a method of vaccination, notably against flavivirus infection, that comprises administration of a flavivirus-like particle to a subject in need thereof. The invention also relates to a vaccine composition comprising a flavivirus-like particle and a pharmaceutically acceptable carrier. The invention further provides an immunogenic composition comprising in a pharmaceutical acceptable carrier, a flavivirus-like particle disclosed herein.

The vaccine and immunogenic compositions of the invention may be drawn to confer immunity, or elicit an immune response against flaviviruses.

However, where the flavivirus-like particles of the invention further carry an additional gene encoding another antigen, different from flavivirus antigens, the invention provides a recombinant viral vaccine useful to raise an immune response against said antigen. Actually, the use of pseudo-particles described herein makes it possible to improve the elicited immune response through combining several presentations and processing pathways of an antigen. For instance, a vaccine composition of the invention, when administered, results in the flavivirus-like particles infecting cells of the host. The transgene encoding the antigen is then integrated in the cellular genome, and subsequently expressed by the cell, such that there is both a cellular and a humoral immune response elicited by the vaccine composition.

Advantageously, the flavivirus-like particles may further carry a transgene encoding an immune modulator, which allows for enhancement of the raised immune reaction.

The vaccination or immunogenic composition of the present invention may additionally contain an adjuvant. A number of adjuvants are known to those skilled in the art. Examples of suitable adjuvants include, for example, include aluminum hydroxide; Saponin; detergents such as Tween 80; animal, mineral or vegetable oils, Corynebacterium or Propionibacterium -derived adjuvants; Mycobacterium bovis (Bacillus Calmette and Guerinn, or BCG); cytokines; acrylic acid polymers such as carbomer; EMA; or combinations thereof.

The route of administration is any conventional route used in the vaccine field. As general guidance, a vaccine composition of the invention is administered via a mucosal surface, *e.g.*, an ocular, intranasal, pulmonary, oral, intestinal, rectal, vaginal, and urinary tract surface; or via a parenteral route, *e.g*., by an intravenous, subcutaneous, intraperitoneal, intradermal, intraepidermal, or intramuscular route. The choice of administration route depends on the formulation that is selected.

In still another embodiment the particles of the invention may be used as vectors for gene transfer and/or gene therapy. Gene therapy is defined as the introduction of genetic material into a cell in order to either change its phenotype or genotype. Owing to their tropism for either primary or cancerous permissive cells, the flavivirus-like particles described herein comprise an efficient gene delivery system amenable to scale up for reproducibly producing large titers of infectious, replication-defective flavivirus-like particles.

Accordingly, the invention relates to a method for *in vivo* or *in vitro* transferring a transgene of interest in a cell, which method comprises infecting a cell with a flavivirus-like particle of the invention, wherein the particle carries a transgene of interest.

The invention further relates to the use of a flavivirus-like particle of the invention, that carries a transgene of interest, for the preparation of a medicament for the prevention or treatment of a disease in a patient, wherein the flavivirus-like particle allows the transfer of the transgene of interest into a cell of the patient, and encodes a product that has a prophylactic or therapeutic effect against the disease.

Preferably, the targeted cell is selected from the group consisting of mammalian mononuclear macrophages, monocytes or dendritic cells, endothelial cells, fibroblasts, neurone/neuroblastoma cells, hepatocyte/hepatoma cells.

The invention will be further understood in view of the following examples and the annexed figures.

### LEGEND TO THE FIGURES :

Figures 1A and 1B show flavivirus prME and retroviral expression constructs. (A) : A cDNA derived from a flavivirus polyprotein gene was used to express the prM and E glycoproteins and the carboxy-terminus of the C protein, which provides the signal peptide for prM (SP prM). The position of the stop codon (star) inserted in the expression constructs to terminate translation of the proteins is shown. The transmembrane domain (TMD) of prM provides the signal peptide (SP E) for the E glycoprotein. NTR, non-translated region;. (**B**) The expression constructs encoding the different components required to assemble infectious pseudo-particles are shown. The filled boxes represent the viral genes and the marker gene (GFP) transferred to the infected cells. The open boxes show the *cis*-acting sequences. LTR, long terminal repeat; CMV, human cytomegalovirus immediate-early promoter; PBS, primer binding site; Ψ, packaging sequence; PPT, poly-purine track; polyA, polyadenylation site. Vector particles were produced by co-transfection of plasmids harboring the packaging functions, the transfer vector and the viral glycoproteins (GP) into 293T cells. The viral GPs were the West Nile Virus, Dengue virus and Yellow fever virus prM and E glycoproteins, expressed a ΔCprME polyprotein (Fig. 6A), the VSV-G or the RD114 glycoproteins. The supernatants of transfected cells were collected during transient expression, concentrated by ultracentrifugation, and used for target cell transduction.

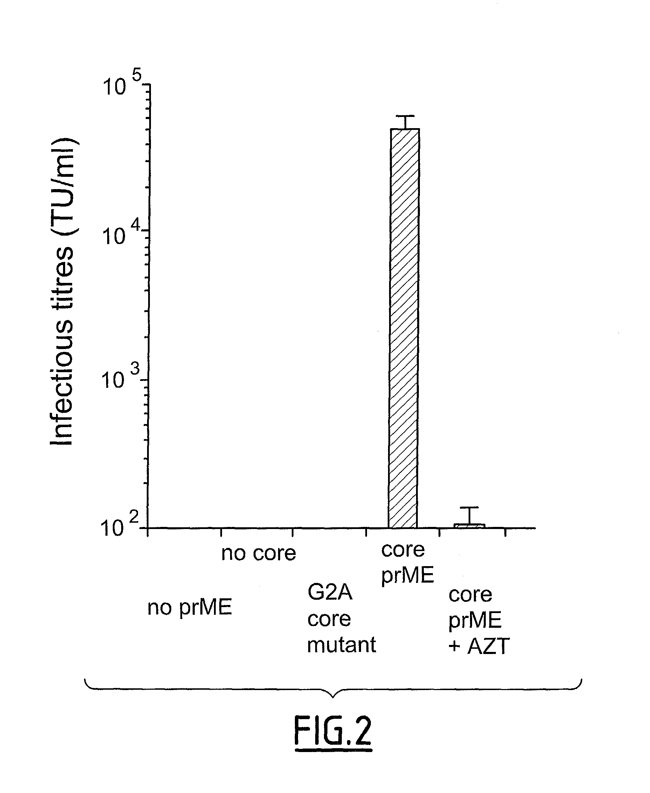

Figure 2 depicts the results of infectivity of FVpp generated without prM and E glycoproteins (no prME), without retroviral core proteins (no core), with MLV-G2A assembly-defective core proteins (G2A core mutant), with prM and E glycoproteins expressed expressed from the same vector in *cis* (core prME). FVpp were treated with 25 µM AZT (3'-azido-3'-deoxythymidine; Sigma-Aldrich, France) before and during infection of target cells (core prME + AZT). Infectious titres, expressed as TU/ml, were determined on Vero target cells and are displayed as mean ± standard deviations of up to four experiments.

### EXAMPLES :

### EXAMPLE 1 : Generation of flavivirus pseudo-particles (FVpp)

Flavivirus pseudo-particles (FVpp) were generated by assembling full-length, unmodified prM and E glycoproteins onto retroviral core proteins derived from murine leukemia virus (MLV) (Figure 1).

### Construction of expression vectors encoding the viral components, i.e., prM, E, or prME glycoproteins and viral core proteins

Plasmids expressing wild type prME polyproteins were constructed by standard methods (Sambrook et *al.,* 1989).

Briefly, the phCMV-ΔC prME-WNV expression vector encoding the prM and E glycoproteins from West Nile Virus was generated by inserting a blunt-ended fragment encoding the last 22 residues of flavivirus core (C) and all of prM and E proteins into the *Bam*Hl digested and Klenow blunted vector phCMV-G (Nègre *et al.,* 2000).

The sequence of the ΔCprME open reading frame of West Nile Virus is shown in SEQ ID No 4, whereas the aminoacid sequence if its ΔCprME polyprotein is shown in SEQ ID No 5.

The sequence of the ΔCprME open reading frame of Dengue Virus is shown in SEQ ID No 6, whereas the aminoacid sequence if its ΔCprME polyprotein is shown in SEQ ID No 7.

The sequence of the ΔCprME open reading frame of Yellow Fever Virus is shown in SEQ ID No 8, whereas the aminoacid sequence if its ΔCprME polyprotein is shown in SEQ ID No 9.

Expression vectors for prME glycoproteins of Yellow Fever Virus were constructed by similar strategies.

Flavivirus prME glycoproteins were therefore expressed from a polyprotein containing the carboxy-terminus of the core (ΔC) protein, which served as signal peptide for the prM glycoproteins whereas the transmembrane domain of prM served as signal peptide for E glycoproteins.

### Generation of flavivirus pseudo-particles

Retroviruses were chosen as platforms for assembly of FVpp because their cores can incorporate a variety of different cellular and viral glycoproteins and because they can easily package and integrate genetic markers into host cell DNA.

FVpp were produced by transfecting 293T human embryo kidney cells (ATCC CRL-1573) with three expression vectors encoding a ΔCprME polyprotein, the MLV core proteins and a packaging-competent MLV-derived genome harbouring the GFP (green fluorescent protein) marker gene. This construct contains the GFP marker gene, whose expression is driven by the CMV (cyto-megalovirus) immediate early promoter. Both CMV and GFP nucleic acid sequences where inserted in a retroviral vector derived from MLV in which the gag, pol and env viral gene where removed and in which the retroviral cis-acting elements that control vector genome packaging, reverse transcription and integration were retained.

Control pseudo-particles were generated with the VSV-G glycoprotein (Nègre *et al.,* 2000), the RD114 virus envelope glycoprotein (Sandrin *et al.,* 2002), and/or with assembly-defective MLV core proteins (MLV-G2A) (Swanstrom et *al*., 1997).

Briefly, expression constructs were transfected into 2.5×10⁶ 293T cells seeded the day before in 10 cm plates using a calcium-phosphate transfection protocol (Clontech, France) according to the manufacturer's recommendations. The medium (8 ml/plate) was replaced 16 hrs after transfection. Supernatants containing the pseudo-particles were harvested 24 hrs later, filtered through 0.45 µm-pore-sized membranes and processed as described before (Nègre *et al.,* 2000).

### Immunoblot analysis of structural components of the pseudo particles

Lysates of transfected cells and of pseudo-particles pelleted through 20% sucrose-cushions were immunobloted. Expression of prM and E glycoproteins from West Nile virus or Dengue Virus or Yellow Fever virus and of MLV core proteins was revealed with monoclonal antibodies against prM and E (gift from P Despres, Institut Pasteur, Paris) and with an anti-capsid (MLV CA) antiserum (ViroMed Biosafety Laboratories, USA), as described previously (Sandrin *et al.,* 2002). VSV-G expressed in control pseudo-particles was detected with the monoclonal antibody P5D4 (Sigma-Aldrich, France).

Analysis of immunoblots of transfected cells showed that the structural components of the pseudo-particles were readily detected at the expected molecular weights; *i.e.* 30 kDa for prM, 60 kDa for E, 60 kDa for VSV-G and 70 kDa for the RD114 glycoprotein. MLV core proteins were detected as a Gag protein precusor of 65 kDa that was partially processed by the MLV protease into mature core components.

The prM and E glycoproteins were readily detected in the pellets of purified virions generated with the wild-type MLV core particles but not with the viral assembly-deficient MLV-G2A mutant.

Comparison of the relative levels of VSV-G or flavivirus glycoproteins in producer cell lysates versus viral pellets suggests efficient incorporation of prM and E into viral particles. Likewise, could prM and E glycoproteins efficiently assemble on retroviral core proteins derived from HIV-1.

Altogether, these results indicate that transient over-expression of prM and E in 293T cells leads to specific and efficient incorporation of flavivirus glycoproteins into pseudo-particles generated with retroviral cores. Since flaviviruses bud from the ER, this implies that FVpp form by budding into the ER lumen or, alternatively, that a fraction of prME to reach the cell surface where MLV budding normally occurs (Swanstrom and Wills, 1997).

### EXAMPLE 2 : FVpp cell line infectivity

Infectivity of FVpp generated with West Nile virus or Dengue virus was assessed on the following target cell lines : Hela human epithelial adenocarcinoma (CCL-2), TE671 human rhabdomyosarcoma (ATCC CRL-8805), and VERO African green monkey kidney (CCL-81).

Target cells (seeded the day before 8×10⁴ cells/well in 12-well plates) were incubated for 3 hrs with dilutions of supernatants from producer cells containing the FVpp, then washed and cultured until expression of the GFP marker gene harboured by the virions was assessed by FACS analysis 72 hrs later (Sandrin *et al.,* 2002). Since the FVpp were generated with replication-defective viral components, this procedure allowed evaluation of the specific infectivity of the pseudo-particles after a one-round infection process.

Infectious titres of up to 5×10⁴ TU/ml were detected for the FVpp generated with West Nile virus on Vero African green monkey cells (Figure 2). Upon concentration of the producer cell supernatants by ultra-centrifugation (Sandrin *et al.,* 2002), infectious titres of 10⁶ TU/ml, on average, could be readily obtained.

Incubation of FVpp and target cells with AZT, a reverse-transcriptase inhibitor that prevents conversion of the retroviral RNA genome of the FVpp as integration-competent proviral DNA, inhibited transduction (Figure 2).

Moreover, long-term expression of GFP could be demonstrated after serial passaging of the infected cells for more than one month.

These results indicated that infection of the target cells led to integration in host cell DNA and stable expression of the GFP marker gene transduced by the FVpp. Additionally, no infection could be obtained with viral particles lacking both prM and E glycoproteins, or lacking core proteins, or, alternatively, when using the MLV-G2A assembly-defective core proteins (Figure 2).

Similar results were obtained with FVpp habouring West Nile virus prM and E glycoproteins on Hela and TE671 cells, as well as with FVpp generated with Dengue virus glycoproteins on Vero, Hela, and TE671 cells.

Altogether, these results demonstrated that FVpp harbouring the prM and E glycoproteins and retroviral core proteins were infectious, leading to retroviral-mediated integration of their vector genome. These data indicate that FVpp closely mimic the early events of infection by wild-type flavivirus.

In conclusion, the invention provides a tool that allows precise investigation of viral assembly of prME glycoproteins (processing, maturation) and their role in cell entry of flaviviruses. No structural modifications of the prME glycoproteins were required for their correct assembly on retroviral and lentiviral cores and to generate high titre infectious FVpp with functional prME glycoproteins. The insertion of a marker gene into the FVpp allowed precise and rapid determination of the infectivity of these pseudo-particles by flow-cytometry.

The development of these functional, infectious flavivirus pseudo-particles make it now possible to investigate early events of flavivirus infection, such as the identification of novel flavivirus receptor(s) or co-receptor(s), to carry out diagnostic assays for the detection of neutralising antibodies in seroconverted patients, and to develop efficient inhibitors to flavivirus infection, such as immunological inhibitors, vaccines and neutralizing antibodies, and pharmacological inhibitors.

### REFERENCES

Ausubel F.M. et *al.* (eds.), Current Protocols in Molecular Biology, John Wiley & Sons, Inc. (1994).

Felgner et *al. Science* **337,** 387-388. (1989).

Guguen-Guillouzo and Guillouzo. Methods for preparation of adult and fetal hepatocytes. p 1-12. In A. Guillouzo and C. Guguen-Guillouzo (ed.), Isolated and cultured hepatocytes. Les Editions INSERM Paris. John Libbey and Co, Ltd., London, United Kingdom (1986).

Hubalek, Z., and J. Halouzka. 1999. West Nile fever―a reemerging mosquito-borne viral disease in Europe. Emerg. Infect. Dis. **5**:643-650

Lanciotti, R. S., A. J. Kerst, R. S. Nasci, M. S. Godsey, C. J. Mitchell, H. M. Savage, N. Komar, N. A. Panella, B. C. Allen, K. E. Volpe, B. S. Davis, and J. T. Roehrig. 2000. Rapid detection of West Nile virus from human clinical specimens, field-collected mosquitoes, and avian samples by a TaqMan reverse transcriptase-PCR assay. J. Clin. Microbiol. **38**:4066-4071.Nakabayashi H. et *al., Cancer Res* **42**, 3858-63. (1982).

Nègre D. et *al., Gene Ther* **7**, 1613-1623 (2000).

Sambrook, J., Fritsch, E. F., and Maniatis, T. *Molecular Cloning: A Laboratory Manual,* 2nd Ed. , Cold Spring Harbor Laboratory, Cold Spring Harbor, NY (1989).

Sandrin V. et *al., Blood* **100**, 823-832 (2002).

Swanstrom R., Wills J. W., in *Retroviruses* J. M. Coffin, S. H. Hughes, H. E. Varmus, Eds. (Cold Spring Harbor Laboratory Press, New York, USA, 1997) pp. 263-334.

Wu *et al.,* (1988) J. Biol. Chem. 263:14621-14624.

Wu *et al.,* (1992) J. Biol. Chem. 267:963-967.

## Claims

1. A method for producing flavivirus-like particles *ex vivo* comprising the steps of:
- providing a first nucleic acid sequence comprising a packaging competent retroviral-derived genome;
- providing a second nucleic acid sequence comprising a cDNA encoding core proteins from said retrovirus;
- providing a third nucleic acid sequence comprising a cDNA encoding a polyprotein comprising successively a flavivirus core protein, and a flavivirus prM protein and/or a flavivirus E protein;
- transfecting host cells with said nucleic acid sequences and maintaining the transfected cells in culture for sufficient time to allow expression of the cDNAs to produce structural proteins from flavivirus and retrovirus; and allowing the structural proteins to form virus-like particles.

2. The method according to claim 1, wherein said packaging competent retroviral-derived genome and core proteins are from a retrovirus selected from the group consisting of MLV, ALV, RSV, MPMV, HIV-1, HIV-2, SIV, EIAV, CAEV, or HFV.

3. The method according to claim 1 or 2, wherein said flavivirus core protein is a carboxy-terminus of flavivirus core that comprises the core protein signal peptide.

4. The method according to any of claims 1 to 3, wherein said polyprotein comprises a flavivirus core protein and a flavivirus prM protein.

5. The method according to any of claims 1 to 4, wherein said polyprotein comprises a flavivirus core protein and a flavivirus E protein.

6. The method according to any of claims 1 to 5, comprising native flavivirus prM and/or E protein.

7. The method according to any of claims 1 to 6, wherein said polyprotein comprises a flavivirus core protein, a native flavivirus prM protein and a native flavivirus E protein.

8. The method according to any of preceding claims, wherein said core, prM and E flavivirus proteins are derived from a same flavivirus.

9. The method according to any of preceding claims, wherein said flavivirus is selected from the group consisting of West Nile Virus, Dengue Virus and Yellow Fever Virus.

10. The method according to any of preceding claims, wherein said nucleic acid sequence comprising a packaging competent retroviral-derived genome further comprises a transgene.

11. An infectious flavivirus-like particle susceptible to be obtained by a method according to any of preceding claims, comprising the core proteins from a retrovirus, and a flavivirus prM glycoprotein and/or a flavivirus E glycoprotein.

12. The infectious particle according to claim 11, comprising prM and E flavivirus glycoproteins.

13. The infectious particle according to claim 11, comprising prM flavivirus glycoprotein.

14. The infectious particle according to claim 11, comprising E flavivirus glycoprotein.

15. The infectious particle according to any of claims 11 to 14, comprising native prM and/or E flavivirus glycoprotein.

16. The infectious particle according to any of claims 11 to 15, wherein said retrovirus is selected from the group consisting of MLV, ALV, RSV, MPMV, HIV-1, HIV-2, SIV, EIAV, CAEV, or HFV.

17. The infectious particle according to any of claims 11 to 16, wherein said flavivirus is selected from the group consisting of West Nile Virus, Dengue Virus and Yellow Fever Virus.

18. The infectious particle according to any of claims 11 to 17, wherein said nucleic acid sequence comprising a packaging competent retroviral-derived genome further comprises a transgene.

19. Use of three nucleic acid sequences for the preparation of a medicament useful as a vaccine against a flavivirus infection, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a flavivirus core protein, and a flavivirus prM protein and/or a flavivirus E protein ;
and, when transferred into cells of a subject, the nucleic acids sequences allow the production of structural proteins from flavivirus and retrovirus, wherein the structural proteins form virus-like particles that are immunogenic.

20. A method for *ex vivo* identification of a receptor for flavivirus prM and/or E glycoprotein comprising detection of the binding of an infectious particle according to any of claims 11 to 18, to a cell receptor.

21. A method for *ex vivo* identifying a cell receptor for a flavivirus comprising the step consisting of:
- transfecting a cell which is not permissive for flavivirus infection with a nucleic acid sequence encoding a protein likely to be a receptor for flavivirus;
- contacting said transformed cell with a flavivirus-like particle according to any of claims 11 to 18;
- determining whether said transformed cell has become permissive or not for flavivirus infection; and
- identifying as a cell receptor for flavivirus said protein expressed by the transformed cell that has become permissive.

22. A method for *ex vivo* identifying a cell receptor for a flavivirus comprising the step consisting of:
- providing an expression cDNA library obtained from a cell permissive for flavivirus infection;
- transfecting cells that are not permissive for flavivirus infection with said expression cDNA library;
- contacting said transformed cells with flavivirus-like particles according to any of claims 11 to 17;
- identifying and isolating those transformed cells that have become permissive for flavivirus infection;
- isolating the expression vector transfected in cells that have become permissive; and
- identifying as a receptor for a flavivirus the proteins encoded by the cDNA sequence of said isolated expression vectors.

23. A method of *ex vivo* screening or identification of molecules capable of interfering with flavivirus entry in cells comprising comparison of the level of cell infection by an infectious particle according to any of claims 11 to 17 in the presence or the absence of a candidate molecule.

24. A method of *in vitro* diagnosis of a flavivirus infection in a patient, comprising detecting immune complexes formed by interaction of anti-flavivirus antibodies likely to be present in a biological sample of the patient with flavivirus-like particle according to any of claims 11 to 17.

25. A method of *in vitro* diagnosis of a flavivirus infection in a patient, comprising detecting an inhibitory effect of anti-flavivirus antibodies likely to be present in a biological sample of the patient, on the infection of a permissive cell by flavivirus-like particles according to any of claims 11 to 17.

26. A diagnostic kit useful for the method of claim 25, comprising a flavivirus-like particle according to any of claim 11 to 17 and appropriate means of detection of said immune complexes.

27. Vaccine composition comprising a flavivirus-like particle according to any of claims 11 to 18 and a pharmaceutically acceptable carrier.

28. A method for *in vitro* transferring a transgene of interest in a target cell comprising infecting a cell with a flavivirus-like particle as described in any of claims 11 to 17, wherein the flavivirus-like particle carries a transgene of interest.

29. Use of a flavivirus-like particle according to any of claims 11 to 17, that carries a transgene of interest, for the preparation of a medicament for the prevention or treatment of a disease in a patient, wherein the flavivirus-like particle allows the transfer of the transgene of interest into a cell of the patient, and encodes a product that has a prophylactic or therapeutic effect against the disease.

30. A transformed host cell that contains :
- a first nucleic acid sequence comprising a packaging competent retrovirus-derived genome;
- a second nucleic acid sequence comprising a cDNA encoding the core proteins from said retrovirus; and
- a third nucleic acid sequence comprising a cDNA encoding a polyprotein comprising successively a flavivirus core protein, and a flavivirus prM protein and/or a flavivirus E protein.

31. A method of *ex vivo* screening or identification of molecules capable of interfering with flavivirus entry in cells comprising comparison the level of transformed host cell fusion to a target host cell, in the presence or the absence of a candidate molecule.

32. The method according to claim 31, comprising the steps consisting of:
- co-culturing a transformed host cell with a target host cell, in the absence or presence of a candidate molecule, under conditions that allow syncytia formation, *i.e.* cell-cell fusion, and flavivirus-like particle entry in target host cell in the absence of any candidate molecule;
- assessing syncytia formation in the absence and in the presence of said candidate molecule;
- comparing syncytia formation measured in presence of said candidate molecule with syncytia formation measured in absence of any candidate molecule;
- identifying as a molecule capable of interfering with flavivirus entry the candidate molecule for which syncytia formation, as measured in the presence of said molecule, is decreased as compared to syncytia formation measured in the absence of any candidate molecule.

33. The method according to any of claims 20 to 25, 28, 31 and 32, wherein said flavivirus is selected from the group consisting of West Nile virus, Dengues virus and Hellow fever virus.


SEQUENCE LISTING

<110> INSERM

<120> Infectious flavivirus pseudo-particles containing functional
prM-E envelope proteins

<130> BET 03-P0161

<160> 9

<170> PatentIn version 3.2

<210> 1
<211> 22
<212> PRT
<213> West Nile virus : 22 C-terminal residues of core protein

<400> 1

Gln Lys Lys Arg Gly Gly Lys Thr Gly Ile Ala Val Met Ile Gly Leu
1               5                   10                  15

Ile Ala Ser Val Gly Ala
            20

<210> 2
<211> 20
<212> PRT
<213> Dengue virus : 20 C-terminal residues of core protein

<400> 2

Met Asn Arg Arg Lys Arg Ser Val Thr Met Leu Leu Met Leu Leu Pro
1               5                   10                  15

Thr Val Leu Ala
            20

<210> 3
<211> 20
<212> PRT
<213> Yellow fever virus : 20 C-terminal residues of core protein

<400> 3

Arg Ser His Asp Val Leu Thr Val Gln Phe Leu Ile Leu Gly Met Leu
1               5                   10                  15

Leu Met Thr Gly
            20

<210> 4
<211> 2070
<212> DNA

<213>  West Nile virus : deltaCprME open reading frame

<400>  4
caaaagaaaa gaggaggaaa gaccggaatt gcagtcatga ttggcctgat cgccagcgta      60

ggagcagtta ccctctctaa cttccaaggg aaggtgatga tgacggtaaa tgctactgac     120

gtcacagatg tcatcacgat tccaacagct gctggaaaga acctatgcat tgtcagagca     180

atggatgtgg gatacatgtg cgatgatact atcacttatg aatgcccagt gctgtcggct     240

ggtaatgatc cagaagacat cgactgttgg tgcacaaagt cagcagtcta cgtcaggtat     300

ggaagatgca ccaagacacg ccactcaaga cgcagtcgga ggtcactgac agtgcagaca     360

cacggagaaa gcactctagc gaacaagaag ggggcttgga tggacagcac caaggccaca     420

aggtatttgg taaaaacaga atcatggatc ttgaggaacc ctggatatgc cctggtggca     480

gccgtcattg gttggatgct tgggagcaac accatgcaga gagttgtgtt tgtcgtgcta     540

ttgcttttgg tggccccagc ttacagcttt aactgccttg gaatgagcaa cagagacttc     600

ttggaaggag tgtctggagc aacatgggtg gatttggttc tcgaaggcga cagctgcgtg     660

actatcatgt ctaaggacaa gcctaccatc gatgtgaaga tgatgaatat ggaggcggcc     720

aacctggcag aggtccgcag ttattgctat ttggctaccg tcagcgatct ctccaccaaa     780

gctgcgtgcc cgaccatggg agaagctcac aatgacaaac gtgctgaccc agcttttgtg     840

tgcagacaag gagtggtgga caggggctgg ggcaacggct gcggactatt tggcaaagga     900

agcattgaca catgcgccaa atttgcctgc tctaccaagg caataggaag aaccatcttg     960

aaagagaata tcaagtacga agtggccatt tttgtccatg gaccaactac tgtggagtcg    1020

cacggaaact actccacaca ggttggagcc actcaggcag ggagattcag catcactcct    1080

gcggcgcctt catacacact aaagcttgga gaatatggag aggtgacagt ggactgtgaa    1140

ccacggtcag ggattgacac caatgcatac tacgtgatga ctgttggaac aaagacgttc    1200

ttggtccatc gtgagtggtt catggacctc aacctccctt ggagcagtgc tggaagtact    1260

gtgtggagga acagagagac gttaatggag tttgaggaac cacacgccac gaagcagtct    1320

gtgatagcat tgggctcaca agagggagct ctgcatcaag ctttggctgg agccattcct    1380

gtggaatttt caagcaacac tgtcaagttg acgtcgggtc atttgaagtg tagagtgaag    1440

atggaaaaat tgcagttgaa gggaacaacc tatggcgtct gttcaaaggc tttcaagttt    1500

cttgggactc ccgcagacac aggtcacggc actgtggtgt tggaattgca gtacactggc    1560

acggatggac cttgcaaagt tcctatctcg tcagtggctt cattgaacga cctaacgcca    1620

gtgggcagat tggtcactgt caaccctttt gtttcagtgg ccacggccaa cgctaaggtc    1680

ctgattgaat tggaaccacc ctttggagac tcatacatag tggtgggcag aggagaacaa    1740

cagattaatc accattggca caagtctgga agcagcattg gcaaagcctt tacaaccacc 1800

ctcaaaggag cgcagagact agccgctcta ggagacacag cttgggactt tggatcagtt 1860

ggaggggtgt tcacctcagt tgggaaggct gtccatcaag tgttcggagg agcattccgc 1920

tcactgttcg gaggcatgtc ctggataacg caaggattgc tgggggctct cctgttgtgg 1980

atgggcatca atgctcgtga taggtccata gctctcacgt ttctcgcagt tggaggagtt 2040

ctgctcttcc tctccgtgaa cgtgcacgct 2070

<210> 5
<211> 694
<212> PRT
<213> West Nile virus : deltaCprME polyprotein

<400> 5

Met Asn Ser Met Gln Lys Lys Arg Gly Gly Lys Thr Gly Ile Ala Val
1               5                   10                  15

Met Ile Gly Leu Ile Ala Ser Val Gly Ala Val Thr Leu Ser Asn Phe
            20                  25                  30

Gln Gly Lys Val Met Met Thr Val Asn Ala Thr Asp Val Thr Asp Val
        35                  40                  45

Ile Thr Ile Pro Thr Ala Ala Gly Lys Asn Leu Cys Ile Val Arg Ala
    50                  55                  60

Met Asp Val Gly Tyr Met Cys Asp Asp Thr Ile Thr Tyr Glu Cys Pro
65                  70                  75                  80

Val Leu Ser Ala Gly Asn Asp Pro Glu Asp Ile Asp Cys Trp Cys Thr
                85                  90                  95

Lys Ser Ala Val Tyr Val Arg Tyr Gly Arg Cys Thr Lys Thr Arg His
            100                 105                 110

Ser Arg Arg Ser Arg Arg Ser Leu Thr Val Gln Thr His Gly Glu Ser
        115                 120                 125

Thr Leu Ala Asn Lys Lys Gly Ala Trp Met Asp Ser Thr Lys Ala Thr
    130                 135                 140

Arg Tyr Leu Val Lys Thr Glu Ser Trp Ile Leu Arg Asn Pro Gly Tyr
145                 150                 155                 160

Ala Leu Val Ala Ala Val Ile Gly Trp Met Leu Gly Ser Asn Thr Met

                165                 170                 175

Gln Arg Val Val Phe Val Val Leu Leu Leu Leu Val Ala Pro Ala Tyr
            180                 185                 190

Ser Phe Asn Cys Leu Gly Met Ser Asn Arg Asp Phe Leu Glu Gly Val
        195                 200                 205

Ser Gly Ala Thr Trp Val Asp Leu Val Leu Glu Gly Asp Ser Cys Val
    210                 215                 220

Thr Ile Met Ser Lys Asp Lys Pro Thr Ile Asp Val Lys Met Met Asn
225                 230                 235                 240

Met Glu Ala Ala Asn Leu Ala Glu Val Arg Ser Tyr Cys Tyr Leu Ala
                245                 250                 255

Thr Val Ser Asp Leu Ser Thr Lys Ala Ala Cys Pro Thr Met Gly Glu
            260                 265                 270

Ala His Asn Asp Lys Arg Ala Asp Pro Ala Phe Val Cys Arg Gln Gly
        275                 280                 285

Val Val Asp Arg Gly Trp Gly Asn Gly Cys Gly Leu Phe Gly Lys Gly
    290                 295                 300

Ser Ile Asp Thr Cys Ala Lys Phe Ala Cys Ser Thr Lys Ala Ile Gly
305                 310                 315                 320

Arg Thr Ile Leu Lys Glu Asn Ile Lys Tyr Glu Val Ala Ile Phe Val
                325                 330                 335

His Gly Pro Thr Thr Val Glu Ser His Gly Asn Tyr Ser Thr Gln Val
            340                 345                 350

Gly Ala Thr Gln Ala Gly Arg Phe Ser Ile Thr Pro Ala Ala Pro Ser
        355                 360                 365

Tyr Thr Leu Lys Leu Gly Glu Tyr Gly Glu Val Thr Val Asp Cys Glu
    370                 375                 380

Pro Arg Ser Gly Ile Asp Thr Asn Ala Tyr Tyr Val Met Thr Val Gly
385                 390                 395                 400

Thr Lys Thr Phe Leu Val His Arg Glu Trp Phe Met Asp Leu Asn Leu
                405                 410                 415

Pro Trp Ser Ser Ala Gly Ser Thr Val Trp Arg Asn Arg Glu Thr Leu
            420                 425                 430

Met Glu Phe Glu Glu Pro His Ala Thr Lys Gln Ser Val Ile Ala Leu
        435                 440                 445

Gly Ser Gln Glu Gly Ala Leu His Gln Ala Leu Ala Gly Ala Ile Pro
    450                 455                 460

Val Glu Phe Ser Ser Asn Thr Val Lys Leu Thr Ser Gly His Leu Lys
465                 470                 475                 480

Cys Arg Val Lys Met Glu Lys Leu Gln Leu Lys Gly Thr Thr Tyr Gly
                485                 490                 495

Val Cys Ser Lys Ala Phe Lys Phe Leu Gly Thr Pro Ala Asp Thr Gly
            500                 505                 510

His Gly Thr Val Val Leu Glu Leu Gln Tyr Thr Gly Thr Asp Gly Pro
        515                 520                 525

Cys Lys Val Pro Ile Ser Ser Val Ala Ser Leu Asn Asp Leu Thr Pro
    530                 535                 540

Val Gly Arg Leu Val Thr Val Asn Pro Phe Val Ser Val Ala Thr Ala
545                 550                 555                 560

Asn Ala Lys Val Leu Ile Glu Leu Glu Pro Pro Phe Gly Asp Ser Tyr
                565                 570                 575

Ile Val Val Gly Arg Gly Glu Gln Gln Ile Asn His His Trp His Lys
            580                 585                 590

Ser Gly Ser Ser Ile Gly Lys Ala Phe Thr Thr Thr Leu Lys Gly Ala
        595                 600                 605

Gln Arg Leu Ala Ala Leu Gly Asp Thr Ala Trp Asp Phe Gly Ser Val
    610                 615                 620

Gly Gly Val Phe Thr Ser Val Gly Lys Ala Val His Gln Val Phe Gly
625                 630                 635                 640

Gly Ala Phe Arg Ser Leu Phe Gly Gly Met Ser Trp Ile Thr Gln Gly
                645                 650                 655

Leu Leu Gly Ala Leu Leu Leu Trp Met Gly Ile Asn Ala Arg Asp Arg
            660                 665                 670

Ser Ile Ala Leu Thr Phe Leu Ala Val Gly Gly Val Leu Leu Phe Leu
        675                 680                 685

Ser Val Asn Val His Ala
    690

<210>  6
<211>  2043
<212>  DNA
<213>  Dengue virus : deltaCprME open reading frame

<400>  6
atgaacagga ggaaaagatc cgtgaccatg ctcctcatgc tgctgcccac agtcctggct      60

ttccatttga ccacacgagg gggagagcca cacatgatag ttagtaagca ggaaagagga     120

aagtcactct tgttcaagac ctctgcaggt gtcaatatgt gcactctcat tgcgatggat     180

ttgggagagt tatgtgagga cacaatgact tacaaatgcc cccggatcac tgaggcggaa     240

ccagatgacg ttgactgctg gtgcaatgcc acagacacat gggtgaccta tgggacgtgt     300

tctcaaaccg gtgaacaccg acgagacaaa cgttccgtgg cactggcccc acacgtggga     360

cttggtctag aaacaagaac cgaaacatgg atgtcctctg aaggcgcctg gaaacaaata     420

caaaaagtgg agacttgggc tttgagacac ccaggattca cggtgatagc tcttttttta     480

gcacatgcca taggaacatc catcactcag aaagggatca ttttcattct gctgatgctg     540

gtaacaccat caatggccat gcgatgcgtg ggaataggca acagagactt cgttgaagga     600

ctgtcaggag caacgtgggt ggacgtggta ttggagcatg gaagctgcgt caccaccatg     660

gcaaaaaata aaccaacatt ggacattgaa ctcttgaaga cggaggtcac gaaccctgcc     720

gtcttgcgca aattgtgcat tgaagctaaa atatcaaaca ccaccaccga ttcaagatgt     780

ccaacacaag gagaggctac actggtggaa gaacaagacg cgaactttgt gtgtcgacga     840

acggttgtgg acagaggctg gggcaatggc tgcggactat ttggaaaagg aagcctactg     900

acgtgtgcta agttcaagtg tgtgacaaaa ctggaaggaa agatagttca atatgaaaac     960

ttaaaatatt cagtgatagt cactgtccac acaggggacc agcaccaggt gggaaacgag    1020

actacagaac atggaacaat tgcaaccata acacctcaag ctcctacgtc ggaaatacag    1080

ttgacagact acggaaccct tacactggac tgctcaccca gaacagggct ggactttaat    1140

gagatggtgc tattgacaat gaaagaaaaa tcatggcttg tccacaaaca atggtttcta    1200

gacttaccac tgccttggac ttcgggggct tcaacatccc aagagacttg gaacagacaa    1260

gatttgctgg tcacattcaa gacagctcat gcaaagaagc aggaagtagt cgtactggga 1320

tcacaggaag gagcaatgca cactgcgttg accggggcga cagaaatcca gacgtcagga 1380

acgacaacaa tctttgcagg acacctgaaa tgcagattaa aaatggataa actgacttta 1440

aaagggatgt catatgtgat gtgcacaggc tcatttaagc tagagaagga agtggctgag 1500

acccagcatg gaactgtcct agtgcaggtt aaatacgaag gaacagatgc gccatgcaag 1560

atcccctttt cgacccaaga tgagaaagga gtgacccaga atgggagatt gataacagcc 1620

aatcccatag ttactgacaa agaaaaacca gtcaacattg agacagaacc accttttggt 1680

gagagctaca tcatagtagg ggcaggtgaa aaagctttga aactaagctg gttcaagaaa 1740

ggaagcagca tagggaaaat gttcgaagca accgcccgag gagcacgaag gatggctatc 1800

ctgggagaca ccgcatggga cttcggctct ataggaggag tgtttacgtc tgtgggaaaa 1860

ttggtacacc aggtttttgg aaccgcatac ggggtcctgt tcagcggcgt ttcttggacc 1920

atgaaaatag gaatagggat tctgctgaca tggttgggat taaattcaag gagcgcgtcg 1980

ctttcgatga cgtgcattgc agttggcatg gttacactgt acctaggagt catggttcaa 2040

gcg 2043

<210> 7
<211> 684
<212> PRT
<213> Dengue virus : deltaCprME polyprotein

<400> 7

Met Asn Ser Met Asn Arg Arg Lys Arg Ser Val Thr Met Leu Leu Met
1               5                   10                  15

Leu Leu Pro Thr Val Leu Ala Phe His Leu Thr Thr Arg Gly Gly Glu
            20                  25                  30

Pro His Met Ile Val Ser Lys Gln Glu Arg Gly Lys Ser Leu Leu Phe
        35                  40                  45

Lys Thr Ser Ala Gly Val Asn Met Cys Thr Leu Ile Ala Met Asp Leu
    50                  55                  60

Gly Glu Leu Cys Glu Asp Thr Met Thr Tyr Lys Cys Pro Arg Ile Thr
65                  70                  75                  80

Glu Ala Glu Pro Asp Asp Val Asp Cys Trp Cys Asn Ala Thr Asp Thr
                85                  90                  95

Trp Val Thr Tyr Gly Thr Cys Ser Gln Thr Gly Glu His Arg Arg Asp

            100                 105                 110

Lys Arg Ser Val Ala Leu Ala Pro His Val Gly Leu Gly Leu Glu Thr
        115                 120                 125

Arg Thr Glu Thr Trp Met Ser Ser Glu Gly Ala Trp Lys Gln Ile Gln
    130                 135                 140

Lys Val Glu Thr Trp Ala Leu Arg His Pro Gly Phe Thr Val Ile Ala
145                 150                 155                 160

Leu Phe Leu Ala His Ala Ile Gly Thr Ser Ile Thr Gln Lys Gly Ile
                165                 170                 175

Ile Phe Ile Leu Leu Met Leu Val Thr Pro Ser Met Ala Met Arg Cys
            180                 185                 190

Val Gly Ile Gly Asn Arg Asp Phe Val Glu Gly Leu Ser Gly Ala Thr
        195                 200                 205

Trp Val Asp Val Val Leu Glu His Gly Ser Cys Val Thr Thr Met Ala
    210                 215                 220

Lys Asn Lys Pro Thr Leu Asp Ile Glu Leu Leu Lys Thr Glu Val Thr
225                 230                 235                 240

Asn Pro Ala Val Leu Arg Lys Leu Cys Ile Glu Ala Lys Ile Ser Asn
                245                 250                 255

Thr Thr Thr Asp Ser Arg Cys Pro Thr Gln Gly Glu Ala Thr Leu Val
            260                 265                 270

Glu Glu Gln Asp Ala Asn Phe Val Cys Arg Arg Thr Val Val Asp Arg
        275                 280                 285

Gly Trp Gly Asn Gly Cys Gly Leu Phe Gly Lys Gly Ser Leu Leu Thr
    290                 295                 300

Cys Ala Lys Phe Lys Cys Val Thr Lys Leu Glu Gly Lys Ile Val Gln
305                 310                 315                 320

Tyr Glu Asn Leu Lys Tyr Ser Val Ile Val Thr Val His Thr Gly Asp
                325                 330                 335

Gln His Gln Val Gly Asn Glu Thr Thr Glu His Gly Thr Ile Ala Thr
            340                 345                 350

Ile Thr Pro Gln Ala Pro Thr Ser Glu Ile Gln Leu Thr Asp Tyr Gly
        355                 360                 365

Thr Leu Thr Leu Asp Cys Ser Pro Arg Thr Gly Leu Asp Phe Asn Glu
    370                 375                 380

Met Val Leu Leu Thr Met Lys Glu Lys Ser Trp Leu Val His Lys Gln
385                 390                 395                 400

Trp Phe Leu Asp Leu Pro Leu Pro Trp Thr Ser Gly Ala Ser Thr Ser
                405                 410                 415

Gln Glu Thr Trp Asn Arg Gln Asp Leu Leu Val Thr Phe Lys Thr Ala
            420                 425                 430

His Ala Lys Lys Gln Glu Val Val Val Leu Gly Ser Gln Glu Gly Ala
        435                 440                 445

Met His Thr Ala Leu Thr Gly Ala Thr Glu Ile Gln Thr Ser Gly Thr
    450                 455                 460

Thr Thr Ile Phe Ala Gly His Leu Lys Cys Arg Leu Lys Met Asp Lys
465                 470                 475                 480

Leu Thr Leu Lys Gly Met Ser Tyr Val Met Cys Thr Gly Ser Phe Lys
                485                 490                 495

Leu Glu Lys Glu Val Ala Glu Thr Gln His Gly Thr Val Leu Val Gln
            500                 505                 510

Val Lys Tyr Glu Gly Thr Asp Ala Pro Cys Lys Ile Pro Phe Ser Thr
        515                 520                 525

Gln Asp Glu Lys Gly Val Thr Gln Asn Gly Arg Leu Ile Thr Ala Asn
    530                 535                 540

Pro Ile Val Thr Asp Lys Glu Lys Pro Val Asn Ile Glu Thr Glu Pro
545                 550                 555                 560

Pro Phe Gly Glu Ser Tyr Ile Ile Val Gly Ala Gly Glu Lys Ala Leu
                565                 570                 575

Lys Leu Ser Trp Phe Lys Lys Gly Ser Ser Ile Gly Lys Met Phe Glu
            580                 585                 590

Ala Thr Ala Arg Gly Ala Arg Arg Met Ala Ile Leu Gly Asp Thr Ala
        595                 600                 605

Trp Asp Phe Gly Ser Ile Gly Gly Val Phe Thr Ser Val Gly Lys Leu
    610                 615                 620

Val His Gln Val Phe Gly Thr Ala Tyr Gly Val Leu Phe Ser Gly Val
625                 630                 635                 640

Ser Trp Thr Met Lys Ile Gly Ile Gly Ile Leu Leu Thr Trp Leu Gly
                645                 650                 655

Leu Asn Ser Arg Ser Ala Ser Leu Ser Met Thr Cys Ile Ala Val Gly
            660                 665                 670

Met Val Thr Leu Tyr Leu Gly Val Met Val Gln Ala
        675                 680

<210> 8
<211> 2043
<212> DNA
<213> Yellow fever virus : deltaCprME open reading frame

<400> 8
atggcacgtt cccatgatgt tctgactgtg caattcctaa ttttgggaat gctgttgatg 60

acgggtggag tgaccttggt gcggaaaaac agatggttgc tcctaaatgt gacatctgag 120

gacctcggga aaacattctc tgtgggcaca ggcaactgca caacaaacat tttggaagcc 180

aagtactggt gcccagactc aatggaatac aactgtccca atctcagtcc aagagaggag 240

ccagatgaca ttgattgctg gtgctatggg gtggaaaacg ttagagtcgc atatggtaag 300

tgtgactcag caggcaggtc taggaggtca agaagggcca ttgacttgcc tacgcatgaa 360

aaccatggtt tgaagacccg gcaagaaaaa tggatgactg gaagaatggg tgaaaggcaa 420

ctccaaaaga ttgagagatg gttcgtgagg aacccctttt ttgcagtgac ggctctgacc 480

attgcctacc ttgtgggaag caacatgacg caacgagtcg tgattgccct actggtcttg 540

gctgttggtc cggcctactc agctcactgc attggaatta ctgacaggga tttcattgag 600

ggggtgcatg gaggaacttg ggtttcagct accctggagc aagacaagtg tgtcactgtt 660

atggcccctg acaagccttc attggacatc tcactagaga cagtagccat tgatagacct 720

gctgaggtga ggaaagtgtg ttacaatgca gttctcactc atgtgaagat taatgacaag 780

tgccccagca ctggagaggc ccacctagct gaagagaacg aaggggacaa tgcgtgcaag 840

cgcacttatt ctgatagagg ctggggcaat ggctgtggcc tatttgggaa agggagcatt 900

gtggcatgcg ccaaattcac ttgtgccaaa tccatgagtt tgtttgaggt tgatcagacc 960

aaaattcagt atgtcatcag agcacaattg catgtagggg ccaagcagga aaattggaat 1020

accgacatta agactctcaa gtttgatgcc ctgtcaggct cccaggaagt cgagttcatt 1080

gggtatggaa aagctacact ggaatgccag gtgcaaactg cggtggactt tggtaacagt 1140

tacatcgctg agatggaaac agagagctgg atagtggaca gacagtgggc ccaggacttg 1200

accctgccat ggcagagtgg aagtggcggg gtgtggagag agatgcatca tcttgtcgaa 1260

tttgaacctc cgcatgccgc cactatcaga gtactggccc tgggaaacca ggaaggctcc 1320

ttgaaaacag ctcttactgg cgcaatgagg gttacaaagg acacaaatga caacaacctt 1380

tacaaactac atggtggaca tgtttcttgc agagtgaaat tgtcagcttt gacactcaag 1440

gggacatcct acaaaatatg cactgacaaa atgttttttg tcaagaaccc aactgacact 1500

ggccatggca ctgttgtgat gcaggtgaaa gtgtcaaaag gagccccctg caggattcca 1560

gtgatagtag ctgatgatct tacagcggca atcaataaag gcattttggt tacagttaac 1620

cccatcgcct caaccaatga tgatgaagtg ctgattgagg tgaacccacc ttttggagac 1680

agctacatta tcgttgggag aggagattca cgtctcactt accagtggca caaagaggga 1740

agctcaatag gaaagttgtt cactcagacc atgaaaggcg tggaacgcct ggccgtcatg 1800

ggagacaccg cctgggattt cagctccgct ggagggttct tcacttcggt tgggaaagga 1860

attcatacgg tgtttggctc tgcctttcag gggctatttg gcggcttgaa ctggataaca 1920

aaggtcatca tgggggcggt acttatatgg gttggcatca acacaagaaa catgacaatg 1980

tccatgagca tgatcttggt aggagtgatc atgatgtttt tgtctctagg agttggcgcc 2040

taa 2043

<210> 9
<211> 680
<212> PRT
<213> Yellow fever virus : deltaCprME polyprotein

<400> 9

Met Ala Arg Ser His Asp Val Leu Thr Val Gln Phe Leu Ile Leu Gly
1               5                   10                  15

Met Leu Leu Met Thr Gly Gly Val Thr Leu Val Arg Lys Asn Arg Trp
            20                  25                  30

Leu Leu Leu Asn Val Thr Ser Glu Asp Leu Gly Lys Thr Phe Ser Val
        35                  40                  45

Gly Thr Gly Asn Cys Thr Thr Asn Ile Leu Glu Ala Lys Tyr Trp Cys

50                  55                  60

Pro Asp Ser Met Glu Tyr Asn Cys Pro Asn Leu Ser Pro Arg Glu Glu
65                  70                  75                  80

Pro Asp Asp Ile Asp Cys Trp Cys Tyr Gly Val Glu Asn Val Arg Val
                85                  90                  95

Ala Tyr Gly Lys Cys Asp Ser Ala Gly Arg Ser Arg Arg Ser Arg Arg
            100                 105                 110

Ala Ile Asp Leu Pro Thr His Glu Asn His Gly Leu Lys Thr Arg Gln
        115                 120                 125

Glu Lys Trp Met Thr Gly Arg Met Gly Glu Arg Gln Leu Gln Lys Ile
    130                 135                 140

Glu Arg Trp Phe Val Arg Asn Pro Phe Phe Ala Val Thr Ala Leu Thr
145                 150                 155                 160

Ile Ala Tyr Leu Val Gly Ser Asn Met Thr Gln Arg Val Val Ile Ala
                165                 170                 175

Leu Leu Val Leu Ala Val Gly Pro Ala Tyr Ser Ala His Cys Ile Gly
            180                 185                 190

Ile Thr Asp Arg Asp Phe Ile Glu Gly Val His Gly Gly Thr Trp Val
        195                 200                 205

Ser Ala Thr Leu Glu Gln Asp Lys Cys Val Thr Val Met Ala Pro Asp
    210                 215                 220

Lys Pro Ser Leu Asp Ile Ser Leu Glu Thr Val Ala Ile Asp Arg Pro
225                 230                 235                 240

Ala Glu Val Arg Lys Val Cys Tyr Asn Ala Val Leu Thr His Val Lys
                245                 250                 255

Ile Asn Asp Lys Cys Pro Ser Thr Gly Glu Ala His Leu Ala Glu Glu
            260                 265                 270

Asn Glu Gly Asp Asn Ala Cys Lys Arg Thr Tyr Ser Asp Arg Gly Trp
        275                 280                 285

Gly Asn Gly Cys Gly Leu Phe Gly Lys Gly Ser Ile Val Ala Cys Ala
    290                 295                 300

Lys Phe Thr Cys Ala Lys Ser Met Ser Leu Phe Glu Val Asp Gln Thr
305                 310                 315                 320

Lys Ile Gln Tyr Val Ile Arg Ala Gln Leu His Val Gly Ala Lys Gln
                325                 330                 335

Glu Asn Trp Asn Thr Asp Ile Lys Thr Leu Lys Phe Asp Ala Leu Ser
            340                 345                 350

Gly Ser Gln Glu Val Glu Phe Ile Gly Tyr Gly Lys Ala Thr Leu Glu
        355                 360                 365

Cys Gln Val Gln Thr Ala Val Asp Phe Gly Asn Ser Tyr Ile Ala Glu
    370                 375                 380

Met Glu Thr Glu Ser Trp Ile Val Asp Arg Gln Trp Ala Gln Asp Leu
385                 390                 395                 400

Thr Leu Pro Trp Gln Ser Gly Ser Gly Gly Val Trp Arg Glu Met His
                405                 410                 415

His Leu Val Glu Phe Glu Pro Pro His Ala Ala Thr Ile Arg Val Leu
            420                 425                 430

Ala Leu Gly Asn Gln Glu Gly Ser Leu Lys Thr Ala Leu Thr Gly Ala
        435                 440                 445

Met Arg Val Thr Lys Asp Thr Asn Asp Asn Asn Leu Tyr Lys Leu His
    450                 455                 460

Gly Gly His Val Ser Cys Arg Val Lys Leu Ser Ala Leu Thr Leu Lys
465                 470                 475                 480

Gly Thr Ser Tyr Lys Ile Cys Thr Asp Lys Met Phe Phe Val Lys Asn
                485                 490                 495

Pro Thr Asp Thr Gly His Gly Thr Val Val Met Gln Val Lys Val Ser
            500                 505                 510

Lys Gly Ala Pro Cys Arg Ile Pro Val Ile Val Ala Asp Asp Leu Thr
        515                 520                 525

Ala Ala Ile Asn Lys Gly Ile Leu Val Thr Val Asn Pro Ile Ala Ser
    530                 535                 540

Thr Asn Asp Asp Glu Val Leu Ile Glu Val Asn Pro Pro Phe Gly Asp
545                 550                 555                 560

Ser Tyr Ile Ile Val Gly Arg Gly Asp Ser Arg Leu Thr Tyr Gln Trp
                565                 570                 575

His Lys Glu Gly Ser Ser Ile Gly Lys Leu Phe Thr Gln Thr Met Lys
            580                 585                 590

Gly Val Glu Arg Leu Ala Val Met Gly Asp Thr Ala Trp Asp Phe Ser
        595                 600                 605

Ser Ala Gly Gly Phe Phe Thr Ser Val Gly Lys Gly Ile His Thr Val
    610                 615                 620

Phe Gly Ser Ala Phe Gln Gly Leu Phe Gly Gly Leu Asn Trp Ile Thr
625                 630                 635                 640

Lys Val Ile Met Gly Ala Val Leu Ile Trp Val Gly Ile Asn Thr Arg
                645                 650                 655

Asn Met Thr Met Ser Met Ser Met Ile Leu Val Gly Val Ile Met Met
            660                 665                 670

Phe Leu Ser Leu Gly Val Gly Ala
        675                 680